# EUROPÄISCHE PATENTSCHRIFT

(11) **EP 3 930 598 B1**
(45) Veröffentlichungstag und Bekanntmachung des Hinweises auf die Patenterteilung: **27.11.2024**
(21) Anmeldenummer: 20710443.1
(22) Anmeldetag: 02.03.2020
(51) Int. Cl.: A61B 17/28

(54) **MEDIZINISCHES INSTRUMENT UND VERFAHREN ZUM HERSTELLEN EINES MEDIZINISCHEN INSTRUMENTS**
MEDICAL INSTRUMENT AND METHOD FOR PRODUCING A MEDICAL INSTRUMENT
INSTRUMENT MÉDICAL ET PROCÉDÉ POUR LA FABRICATION D'UN INSTRUMENT MÉDICAL

(30) Priorität: 01.03.2019 DE 102019105268
(43) Veröffentlichungstag der Anmeldung: 05.01.2022
(73) Patentinhaber: Aesculap AG, 78532 Tuttlingen (DE)
(72) Erfinder: BARTHELMES, Sven, 78576 Emmingen-Liptingen (DE); FAITSCH, Dominic, 78579 Neuhausen (DE); SCHABERT, Stefanie, 78554 Aldingen (DE)
(74) Vertreter: Hoeger, Stellrecht & Partner Patentanwälte mbB
(86) Internationale Anmeldenummer: PCT/EP2020/055431
(87) Internationale Veröffentlichungsnummer: WO 2020/178235

(56) Entgegenhaltungen:
- EP-A1- 0 416 296
- EP-A2- 2 093 002
- CN-A- 107 962 391
- CN-A- 108 015 599
- DE-C1- 3 506 866

## Beschreibung

Die vorliegende Erfindung betrifft ein medizinisches Instrument mit mindestens einem Instrumentenkörperteil, wobei der mindestens eine Instrumentenkörperteil aus einem Instrumentenkörperteilrohling durch Umformen ausgebildet ist.

Ferner betrifft die vorliegende Erfindung ein Verfahren zum Herstellen eines medizinischen Instruments, welches mindestens ein Instrumentenkörperteil umfasst, bei welchem Verfahren ein Instrumentenkörperteilrohling umgeformt wird und beim Umformen, insbesondere durch Toleranz- und Prozessschwankungen, ausgebildete Grate entfernt werden.

Bekannt sind insbesondere medizinische Instrumente mit zwei verschwenkbar aneinander gelagerten Instrumentenkörperteilen. Die Instrumentenkörperteile derartiger Instrumente werden insbesondere durch Umformen aus einem Rohling, nachfolgend auch als Instrumentenkörperteilrohling bezeichnet, ausgebildet. Dabei bilden sich überstehende oder vorstehende Grate. Diese Grate werden herkömmlich durch handgeführtes Bandschleifen entfernt.

Nachteilig bei der Herstellung bekannter medizinischer Instrumente ist insbesondere, dass durch handgeführte Bearbeitungsprozesse Form- und Maßschwankungen auftreten können. Zudem kann es zu einem undefinierten Hitzeeintrag in das aus dem Instrumentenkörperteilrohling umgeformte Instrumentenkörperteil kommen, wenn die beim Umformen entstehenden Grate durch Bandschleifen, insbesondere handgeführt, entfernt werden.

Aus der EP 0 416 296 A1 ist ein Verfahren zum Herstellen von im Gesenk geprägten oder geschmiedeten Werkstücken bekannt. Eine Maschine zur Herstellung von Zangen ist in der CN 107 962 391 A beschrieben. Die CN 108 015 599 A offenbart ein Verfahren für einen kontinuierlichen Fräsprozess zum Ausbilden innerer und äußerer profilierter Zahnprofile an einem Zangenkopf. Verfahren zum optimierten endkonturnahen Fräsen sind aus der EP 2 093 002 A2 bekannt. Eine Steuerungsvorrichtung für das automatische Entgraten von Werkstücken durch Fräsen ist in der DE 35 06 866 C1 beschrieben.

Es ist daher eine Aufgabe der vorliegenden Erfindung, die Herstellung medizinischer Instrumente der eingangs beschriebenen Art zu verbessern.

Diese Aufgabe wird bei einem medizinischen Instrument der eingangs beschriebenen Art erfindungsgemäß dadurch gelöst, dass das mindestens eine Instrumentenkörperteil mindestens eine durch Fräsen, insbesondere durch maschinelles Fräsen, ausgebildete Entgratungsfläche aufweist, dass die mindestens einen Entgratungsfläche einen oder mehrere Abschnitte umfasst, dass jeder Abschnitt der mindestens einen Entgratungsfläche senkrecht zu einer gemeinsamen Bearbeitungsebene verläuft, dass eine Normale zur Bearbeitungsebene eine Entgratungslängsachse definiert, dass die mindestens eine Entgratungsfläche parallel zur Entgratungslängsachse verläuft, dass das medizinische Instrument in Form einer Schere, einer Zange, eines Nadelhalters oder einer Klemme ausgebildet ist und zwei Instrumentenkörperteile umfasst, dass die zwei Instrumentenkörperteile um eine Schwenkachse verschwenkbar aneinander gelagert sind und dass die Schwenkachse die Entgratungslängsachse definiert.

Durch das wie beschrieben definierte Ausbilden der Entgratungsfläche können Instrumentenkörperteile medizinischer Instrumente hochpräzise und reproduzierbar ausgebildet werden. Insbesondere dann, wenn das Fräsen maschinell erfolgt, insbesondere computergesteuert, können so Prozessschwankungen, die sich bei handgeführten Bearbeitungsprozessen von Instrumentenkörperteilen ergeben und somit unvermeidlich sind, eliminiert oder zumindest signifikant verringert werden. Zudem hat das Fräsen insbesondere den Vorteil, dass es zu keiner übermäßigen Erhitzung des umgeformten Instrumentenkörperteilrohlings kommen kann, wie beim Bearbeiten desselben zum Entfernen der Grate mittels Bandschleifen. Außerdem ergibt sich eine am Instrument beziehungsweise dem mindestens einen Instrumentenkörperteil gut sichtbare Entgratungsfläche, die auch noch nach weiteren optionalen Bearbeitungsschritten wie beispielsweise Polieren oder Strahlen gut sichtbar ist. Eine derartige Entgratungsfläche mit einer wie vorgeschlagen definierten Ausrichtung ist mittels handgeführter Bearbeitungsprozesse so nicht realisierbar. Insgesamt können so medizinische Instrumente mit hoher Qualität und deutlich verbesserter Reproduzierbarkeit ausgebildet werden. Günstig ist es, dass das medizinische Instrument zwei Instrumentenkörperteile umfasst und dass die zwei Instrumentenkörperteile um eine Schwenkachse verschwenkbar aneinander gelagert sind. Diese Ausgestaltung ermöglicht insbesondere die Ausbildung medizinischer Instrumente, beispielsweise in Form von Scheren, Klemmen, Nadelhaltern oder dergleichen. Insbesondere können zwei relativ zueinander bewegliche und zusammenwirkende Werkzeugelemente am Instrument vorgesehen werden, wobei beispielsweise jedes der beiden Instrumentenkörperteile ein Werkzeugelement umfassen oder tragen kann. Die Herstellung des medizinischen Instruments wird insbesondere dadurch vereinfacht, dass die Schwenkachse die Entgratungslängsachse definiert. Beispielsweise kann die Schwenkachse definiert werden durch einen Gelenkstift, der an einem der beiden Instrumentenkörperteile angeordnet oder ausgebildet ist. Das andere der beiden Instrumentenkörperteile kann dann beweglich am Gelenkstift gelagert oder gehaltert werden. Beispielsweise kann ein solcher Gelenkstift in eine Bohrung der Instrumentenkörperteile eingesetzt sein, wobei der Gelenkstift dann mit einem der beiden Instrumentenkörperteile dauerhaft fest verbunden wird, beispielsweise durch Kleben oder Schweißen. Die besondere Ausrichtung der Schwenkachse derart, dass sie durch die Entgratungslängsachse definiert wird, also senkrecht zur Bearbeitungsebene verläuft, hat insbesondere den Vorteil, dass eine solche Bohrung für einen Gelenkstift an den Instrumentenkörperteilen, insbesondere dann auf einfache Weise ausgebildet werden kann, wenn das Instrumentenkörperteil gehalten ist und das Fräswerkzeug relativ zum Instrumentenkörperteil bewegt wird. So können in einem Schritt sowohl die Entgratungsfläche als auch eine die Schwenkachse definierende Bohrung an einem oder beiden Instrumentenkörperteilen ausgebildet werden. Insbesondere lässt sich an einer derartigen exakten Ausrichtung der Schwenkachse des Instruments relativ zur mindestens einen Entgratungsfläche sehr gut erkennen, dass das Instrumentenkörperteil nicht durch einen handgeführten Bearbeitungsprozess entgratet wurde, sondern maschinell.

Vorteilhaft ist es, wenn die mindestens eine Entgratungsfläche in sich geschlossen ausgebildet ist. Eine solche Fläche kann in einem Bearbeitungsschritt ausgebildet werden durch Abfahren des umgeformten Instrumentenkörperteilrohlings mit einem Fräswerkzeug. Insbesondere kann dies auf einfache Weise durch computerunterstütztes Fräsen erreicht werden. Bei Instrumentenkörperteilen können in sich geschlossene Entgratungsflächen insbesondere im Bereich von Fingerringen, die auch als Augen bezeichnet werden, ausgebildet werden.

Vorteilhaft ist es, wenn die mindestens eine Entgratungsfläche abschnittsweise eben und/oder abschnittsweise vom mindestens einen Instrumentenkörperteil weg weisend konvex gekrümmt und/oder vom mindestens einen Instrumentenkörperteil weg weisend konkav gekrümmt ausgebildet ist. Eine solche Entgratungsfläche kann insbesondere stets, also jeder Abschnitt derselben, senkrecht zur Bearbeitungsebene und damit parallel zur Entgratungslängsachse ausgebildet werden. Eine verbesserte Reproduzierbarkeit und eine definierte Ausbildung der mindestens einen Entgratungsfläche sind auf diese Weise möglich. Zudem lassen sich beliebige Krümmungen der mindestens einen Entgratungsfläche realisieren.

Günstig ist es, wenn die mindestens eine abschnittsweise gekrümmte Entgratungsfläche nur eindimensional gekrümmt ausgebildet ist und dass ein Krümmungsradius der Entgratungsfläche ausschließlich bezogen auf die Entgratungslängsachse definiert ist. Mit anderen Worten lässt sich die mindestens eine abschnittsweise gekrümmte Entgratungsfläche als bandförmige Fläche beschreiben, die senkrecht zur Bearbeitungsebene und somit parallel zur Entgratungslängsachse verläuft.

Vorzugsweise ist der Krümmungsradius abschnittsweise konstant oder ändert sich längs der Erstreckung der mindestens einen Entgratungsfläche abschnittsweise oder kontinuierlich. So lassen sich beliebig gekrümmte, insbesondere eindimensional beliebig gekrümmte, Entgratungsflächen ausbilden.

Gemäß einer weiteren bevorzugten Ausführungsform der Erfindung kann vorgesehen sein, dass mindestens zwei Flächenabschnitte der mindestens einen Entgratungsfläche parallel oder im Wesentlichen parallel zueinander verlaufen. Beispielsweise können zwei Entgratungsflächen ebene Flächenabschnitte definieren, die parallel zueinander verlaufen. Insbesondere kann dies an langgestreckten Branchen, auch als Arme medizinischer Instrumente bezeichnet, realisiert werden. Derartige Abschnitte können insbesondere bei medizinischen Instrumenten, die zwei verschwenkbar aneinander gehaltene Instrumentenkörperteile umfassen, proximalseitig eines die Instrumentenkörperteile verbindenden Gelenks oder Gelenkbereichs, welcher auch als Schlussbereich bezeichnet wird, ausgebildet sein. Insbesondere können sie sich zwischen dem Schlussbereich und Fingerringen beziehungsweise Augen der Instrumentenkörperteile erstrecken.

Auf einfache Weise lässt sich ein medizinisches Instrument ausbilden, wenn das mindestens eine Instrumentenkörperteil durch Kaltmassivumformen aus dem Instrumentenkörperteilrohling ausgebildet ist. Zudem lassen sich so sehr stabile Instrumentenkörperteile ausbilden.

Um eine Verletzungsgefahr für einen Nutzer des Instruments zu verringern, ist es vorteilhaft, wenn das mindestens eine Instrumentenkörperteil poliert ist. Insbesondere kann es durch Gleitschleifen poliert sein. So können letzte Ecken und Kanten in definierter Weise entfernt oder verrundet werden.

Um dem medizinischen Instrument eine charakteristische Oberfläche zu verleihen, ist es günstig, dass das mindestens eine Instrumentenkörperteil gestrahlt ist. Insbesondere kann es durch Sandstrahlen oder Kugelstrahlen gestrahlt sein.

Um ein besonders stabiles medizinisches Instrument ausbilden zu können, ist es günstig, wenn das mindestens eine Instrumentenkörperteil aus einem metallischen Werkstoff ausgebildet ist. Insbesondere kann es aus einem Instrumentenstahl ausgebildet sein.

Die eingangs gestellte Aufgabe wird ferner bei einem Verfahren der eingangs beschriebenen Art erfindungsgemäß dadurch gelöst, dass die Grate durch Fräsen, insbesondere maschinelles Fräsen, entfernt werden, dass das mindestens eine Instrumentenkörperteil beim Fräsen gehalten wird, dass das Fräswerkzeug zum Entfernen der Grate um eine Entgratungslängsachse rotiert und zum Ausbilden von mindestens einer Entgratungsfläche in einer Bearbeitungsebene, deren Normale die Entgratungslängsachse definiert, bewegt wird, ohne eine Ausrichtung der Entgratungslängsachse relativ zur Bearbeitungsebene beim Entgraten zu verändern, dass am mindestens einen Instrumentenkörperteil eine Gelenkbohrung ausgebildet wird mit einer Bohrungslängsachse, die parallel oder im Wesentlichen parallel zur Entgratungslängsachse verläuft und dass mindestens zwei, insbesondere nur zwei, Instrumentenkörperteile miteinander um die Bohrungslängsachse verschwenkbar miteinander gekoppelt werden und dass das medizinische Instrument in Form einer Schere, einer Zange, eines Nadelhalters oder einer Klemme ausgebildet wird.

Bei dem beschriebenen Verfahren werden also Grate nicht wie aus dem Technik bekannt durch handgeführtes Bandschleifen entfernt, sondern durch Fräsen. Ferner wird das Fräsen auf besondere Weise durchgeführt, indem das Fräswerkzeug um die Entgratungslängsachse rotiert wird. Die Bearbeitungsebene verläuft somit senkrecht zur Entgratungslängsachse. Der umgeformte Instrumentenkörperteilrohling wird beim gesamten Fräsprozess insbesondere in definierter Weise relativ zum Fräswerkzeug gehalten, und zwar vorzugsweise so, dass die Bearbeitungsebene sich weder relativ zur Entgratungslängsachse noch zum Instrumentenkörperteilrohling in Ihrer Ausrichtung verändert. Auf diese Weise ist es insbesondere möglich, einen Instrumentenkörperteil wie oben beschrieben auszubilden, bei dem jeder Abschnitt der mindestens einen Entgratungsfläche senkrecht zur gemeinsamen Bearbeitungsebene verläuft, wobei die Normale zur Bearbeitungsebene die Entgratungslängsachse definiert, sodass die mindestens eine Entgratungsfläche parallel zur Entgratungslängsachse verläuft. Der Instrumentenkörperteilrohling kann also beispielsweise in eine Maschine eingespannt werden, um ihn zu halten, wobei dann das Fräswerkzeug in der definierten Ausrichtung am Instrumentenkörperteilrohling entlanggeführt wird. Dies kann beispielsweise handgeführt mit Unterstützung einer Führungseinrichtung erfolgen oder vollständig automatisch mit einer CNC-Fräse. Das Entgraten des mindestens einen Instrumentenkörperteils kann auf diese Weise voll automatisch maschinell erfolgen. Günstig ist es, dass am mindestens einen Instrumentenkörperteil eine Gelenkbohrung ausgebildet wird mit einer Bohrungslängsachse, die parallel oder im Wesentlichen parallel zur Entgratungslängsachse verläuft. Dies kann insbesondere auf einfache Weise erreicht werden, wenn der umgeformte Instrumentenkörperteil, der zum Entgraten gehalten wird, mit der Bohrung versehen wird. So kann insbesondere eine hochpräzise Ausrichtung der Bohrungslängsachse, die insbesondere die Schwenkachse eines zwei Instrumentenkörperteile umfassenden medizinischen Instruments definiert, relativ zur mindestens einen Entgratungsfläche erreicht werden. Insbesondere ist eine solche Ausrichtung ein untrügliches Indiz dafür, dass ein medizinisches Instrument gemäß dem vorgeschlagenen Verfahren ausgebildet wurde. Gemäß der Erfindung werden mindestens zwei, insbesondere nur zwei, Instrumentenkörperteile miteinander um die Bohrungslängsachse verschwenkbar miteinander gekoppelt. So lassen sich insbesondere Scheren, Zangen, Nadelhalter, Klemmen oder dergleichen auf einfache Weise ausbilden.

Günstigerweise wird das Entfernen der Grate computergesteuert durchgeführt. So kann insbesondere eine besonders hohe Reproduzierbarkeit bei der Ausbildung von Instrumentenkörperteilen erreicht werden. Ein computergesteuertes Fräsen zum Entfernen der Grate hat zudem den Vorteil, dass keine aufwendigen Führungseinrichtungen erforderlich sind. Eine computergesteuerte Fräse kann insbesondere beliebig programmiert werden, um so beliebige Entgratungsflächen auszubilden.

Vorzugsweise wird beim Entfernen der Grate ein Teil des Instrumentenkörperteils zur Ausbildung der mindestens einen Entgratungsfläche mit entfernt. Dies ist insbesondere so zu verstehen, dass nicht nur beim Umformen des im Instrumentenkörperteilrohlings ausgebildete Grate entfernt werden, sondern auch noch ein Teil des Instrumentenkörperteils mit. So können Instrumentenkörperteile in hoch präziser Form und hoch definiert ausgebildet werden. Zudem lässt sich so eine Breite der mindestens einen Entgratungsfläche in einer Weise vorsehen, dass Sie von einem Anwender gut erkennbar ist. So lassen sich insbesondere Instrumente, die nach dem beschriebenen Fertigungsverfahren hergestellt werden, einfach und sicher von medizinischen Instrumenten unterscheiden, die mittels handgeführter Bearbeitungsprozesse entgratet wurden.

Einfach und kostengünstig lässt sich ein medizinisches Instrument ausbilden, wenn der Instrumentenkörperteilrohling aus einer Platte durch Stanzen oder Schneiden ausgebildet wird. Insbesondere kann ein Instrumentenkörperteilrohling durch Laserschneiden aus einer Platte herausgetrennt werden. So lassen sich insbesondere in definierter und reproduzierbarer Weise Instrumentenkörperteilrohlinge ausbilden.

Besonders stabile medizinische Instrumente mit langer Standzeit können insbesondere dadurch ausgebildet werden, dass das mindestens eine Instrumentenkörperteil aus einem metallischen Werkstoff ausgebildet wird. Insbesondere kann es aus einem Instrumentenstahl ausgebildet werden.

Günstigerweise wird der Instrumentenkörperteilrohling durch Kaltmassivumformen umgeformt. Insbesondere kann dies erreicht werden durch Einlegen des Instrumentenkörperteilrohlings in Pressformwerkzeuge. Der Instrumentenkörperteilrohling kann insbesondere so bemessen werden, dass ein umlaufender oder im Wesentlichen umlaufender Grat beim Umformen ausgebildet wird. Dies erleichtert insbesondere die maschinelle Bearbeitung des Instrumentenkörperteils nach dem Umformen zum Entfernen der Grate. Insbesondere können so definierte Entgratungsflächen ausgebildet werden.

Günstig ist es, wenn der Instrumentenkörperteilrohling auf eine Fertigkontur des mindestens einen Instrumentenkörperteils umgeformt wird, wobei die ausgebildeten Grate über die Fertigkontur vorstehen. Diese Vorgehensweise kann insbesondere sicherstellen, dass die gewünschte Fertigkontur des Instrumentenkörperteils eingehalten werden kann, auch nach dem Entfernen der Grate.

Ferner kann es vorteilhaft sein, wenn beim Umformen des Instrumentenkörperteilrohlings mindestens ein zusammenhängender Grat ausgebildet wird. Ein solcher Grat kann durch eine Abfahrbewegung des Fräswerkzeugs in einem Bearbeitungsgang entfernt werden.

Vorteilhaft ist es, wenn zum verschwenkbaren Koppeln der zwei Instrumentenkörperteile ein gemeinsamer Gelenkstift in die Gelenkbohrungen beider Instrumentenkörperteile eingesetzt und mit einem der beiden Instrumentenkörperteile dauerhaft verbunden wird. Insbesondere kann der Gelenkstift kraft- und/oder form- und/oder stoffschlüssig mit einem der beiden Instrumentenkörperteile verbunden werden. Beispielsweise kann eine Verbindung durch Kleben oder Schweißen erfolgen. So kann insbesondere eine dauerhafte und definierte bewegliche Kopplung der zwei Instrumentenkörperteile miteinander erreicht werden.

Um die Gefahr von Verletzungen beim Handhaben des medizinischen Instruments zu minimieren, ist es günstig, wenn das mindestens eine Instrumentenkörperteil poliert wird. Insbesondere kann es durch Gleitschleifen poliert werden. So können minimale Kanten und verbliebene Grate entfernt werden, insbesondere im Übergangsbereich zur mindestens einen Entgratungsfläche. Zudem kann ein optischer Gesamteindruck des medizinischen Instruments auf diese Weise verbessert werden.

Um dem medizinischen Instrument eine charakteristische Oberfläche zu verleihen, ist es vorteilhaft, wenn das mindestens eine Instrumentenkörperteil nach dem Polieren gestrahlt wird. Insbesondere kann es durch Sandstrahlen oder Kugelstrahlen bearbeitet werden.

Ferner wird die Verwendung eines der oben beschriebenen Verfahren zum Herstellen eines der oben beschriebenen medizinischen Instrumente vorgeschlagen.

Die nachfolgende Beschreibung bevorzugter Ausführungsformen der Erfindung dient im Zusammenhang mit der Zeichnung der näheren Erläuterung. Es zeigen:
- Figur 1:: eine schematische Darstellung des Ausstanzens eines Ausführungsbeispiels eines Instrumentenkörperteilrohlings aus einer Platte mit einem Stanzwerkzeug;
- Figur 2:: eine Schnittansicht längs Linie 2-2 in Figur 1;
- Figur 3:: eine schematische Darstellung eines Ausführungsbeispiels eines aus einem Instrumentenkörperteilrohling umgeformten Instrumentenkörperteils beim Entnehmen aus zusammenwirkenden Umformwerkzeugen;
- Figur 4:: eine Schnittansicht längs Linie 4-4 in Figur 3 beim Einlegen des Instrumentenkörperteilrohlings in die Umformwerkzeuge;
- Figur 5:: eine Ansicht analog Figur 4 nach dem Umformen des Instrumentenkörperteilrohlings durch die zusammenwirkenden Umformwerkzeuge;
- Figur 6:: eine Draufsicht auf das in Figur 3 dargestellte Ausführungsbeispiel eines Instrumentenkörperteils;

- Figur 7:: eine Ansicht des Instrumentenkörperteils aus Figur 6 in Richtung des Pfeils A;
- Figur 8:: eine schematische Darstellung des Instrumentenkörperteils aus den Figuren 6 und 7 beim maschinellen Entgraten mit einem Fräswerkzeug zur Ausbildung von Entgratungsflächen;
- Figur 9:: eine Schnittansicht längs Linie 9-9 in Figur 8;
- Figur 10:: eine Schnittansicht analog Figur 9 nach dem Ausbilden eines Durchsteckschlusskastens sowie einer Gelenkbohrung am Instrumentenkörperteil;
- Figur 11:: eine schematische Darstellung des entgrateten Instrumentenkörperteils beim Polieren; und
- Figur 12:: eine perspektivische Gesamtansicht eines Ausführungsbeispiels eines medizinischen Instruments mit zwei verschwenkbar aneinander gelagerten Instrumentenkörperteilen.

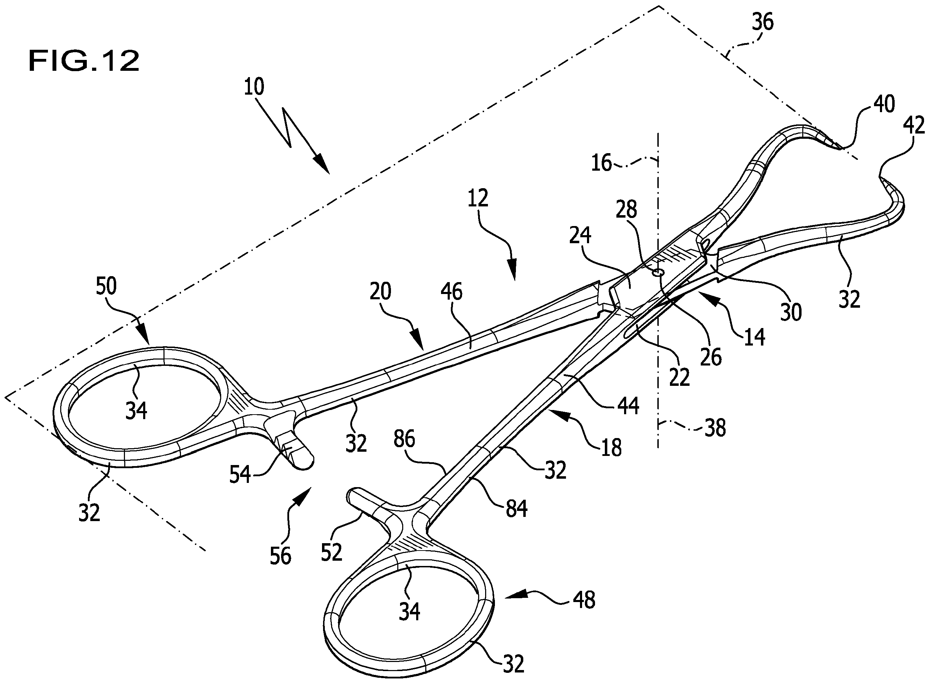

Figur 12 zeigt schematisch eine perspektivische Gesamtansicht eines Ausführungsbeispiels eines medizinischen Instruments 10 in Form einer Tuchklemme 12.

Das Instrument 10 umfasst zwei in einem Schlussbereich 14 um eine Schwenkachse 16 verschwenkbar aneinander gekoppelte Instrumentenkörperteile 18 und 20.

Der Schlussbereich 14 ist in Form eines Durchsteckschlusses ausgebildet mit einem einen Schlitz 22 aufweisenden, im Wesentlichen quaderförmigen Schlusskasten 24. Am Schlusskasten 24 ist ein den Schlitz 22 durchsetzender Gelenkstift 26, dessen Längsachse die Schwenkachse 16 definiert, in eine ebenfalls die Schwenkachse definierende Gelenkbohrung 28 eingesetzt und unbeweglich am Schlusskasten 24 befestigt.

An einem Schlussabschnitt 30 des Instrumentenkörperteils 20 ist ebenfalls eine in den Figuren nicht dargestellte Gelenkbohrung ausgebildet, die der Gelenkstift 26 durchsetzt, wenn der Schlussabschnitt 30 den Schlitz 22 durchgreift.

Die Instrumentenkörperteile 18 und 20 weisen jeweils Entgratungsflächen 32 und 34 auf. Alle Entgratungsflächen 32 und 34 verlaufen parallel zur Schwenkachse 16 und damit senkrecht zu einer Bearbeitungsebene 36. Die Schwenkachse 16 definierte eine Normale 38 zur Bearbeitungsebene 36.

Distale Enden der Instrumentenkörperteile 18, 20 bilden Werkzeugelemente 40 beziehungsweise 42. Bei der Tuchklemme sind diese in Form aufeinander zu weisender Spitzen ausgebildet. Sie können jedoch auch in Form von Schneiden oder stumpfen Klemmelementen ausgebildet sein, um ein Instrument 10 in Form einer Schere oder einer stumpfen Klemme oder eines Nadelhalters auszubilden.

Vom Schlussbereich 14 in proximaler Richtung erstrecken sich Branchen 44 beziehungsweise 46 bis zu in sich geschlossenen ausgebildeten Fingerringen 48 beziehungsweise 50.

Direkt distalseitig der Fingerringe 48 und 50 sind an den Branchen 44 und 46 jeweils verzahnte Sperrelemente 52 beziehungsweise 54 ausgebildet, die zusammen eine Sperreinrichtung 56 bilden, um das Instrument 10 in einem definierten Schließzustand zu halten.

Die Entgratungsflächen 34 im Bereich der Fingerringe 48 und 50 sind vom jeweiligen Fingerring 48 beziehungsweise 50 weg weisend konkav gekrümmt und in sich geschlossen ausgebildet. Ein Krümmungsradius der Entgratungsflächen 34 ändert sich kontinuierlich. Optional könnten die Fingerringe 48 und 50, die bei dem in Figur 12 dargestellten Ausführungsbeispiel in Draufsicht eine ovale Form aufweisen, auch kreisringförmig ausgebildet sein und einen konstanten Krümmungsradius aufweisen.

Flächenabschnitte 84 und 86 der Entgratungsflächen 32 im Bereich der Branchen 44 und 46 zwischen dem Schlussbereich 14 und den Sperrelementen 52 beziehungsweise 54 sind eben oder im Wesentlichen eben ausgebildet und verlaufen zumindest abschnittsweise parallel beziehungsweise im Wesentlichen parallel zueinander.

Die Entgratungsflächen 32 im Bereich der Fingerringe 48 und 50 sind vom jeweiligen Instrumentenkörperteil 18 beziehungsweise 20 weg weisend konvex gekrümmt.

Wie bereits erläutert umfasst jede Entgratungsfläche 32 beziehungsweise 34 einen oder mehrere Abschnitte, wobei alle Abschnitte der Entgratungsflächen 32 und 34 senkrecht zur gemeinsamen Bearbeitungsebene 36 verlaufen.

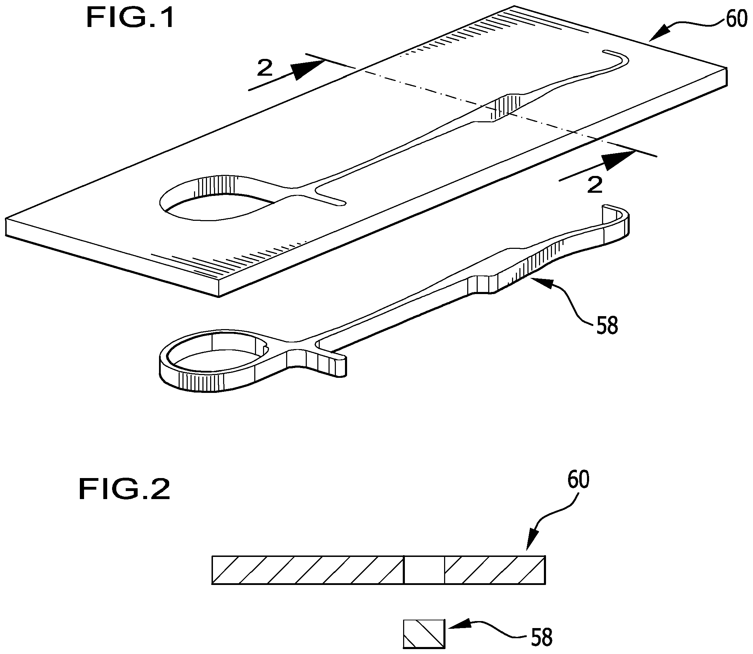

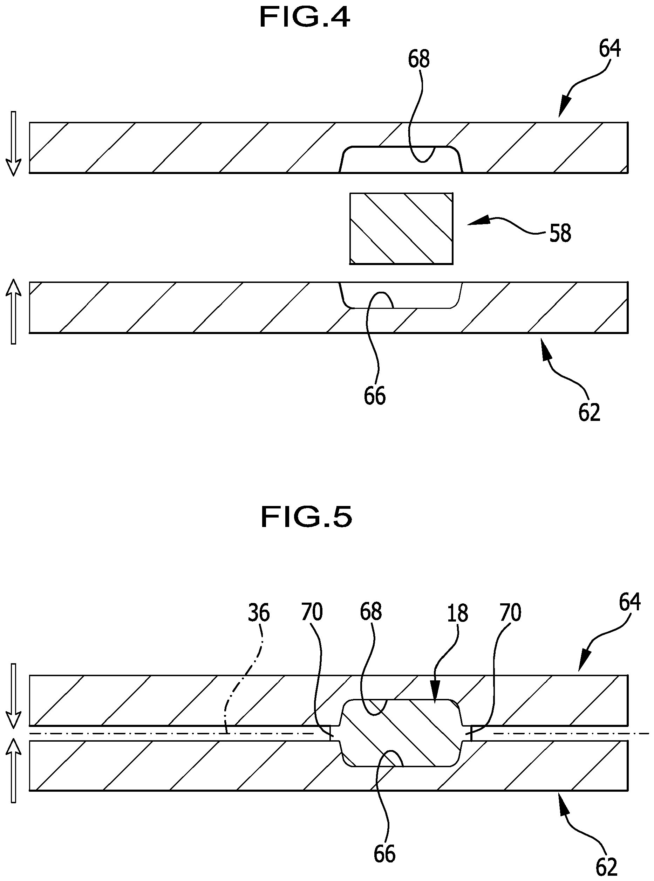

Die Herstellung des Instruments 10 wird nachfolgend in Verbindung mit den Figuren 1 bis 12 näher erläutert.

Zur Ausbildung der Instrumentenkörperteile 18 und 20 werden Instrumentenkörperteilrohlinge 58 umgeformt.

Figur 1 zeigt beispielhaft einen durch Stanzen oder Laserschneiden aus einer Platte 60 ausgestanzten beziehungsweise ausgeschnittenen Instrumentenkörperteilrohling 58, aus dem sowohl der Instrumentenkörperteil 18 als auch der Instrumentenkörperteil 20 ausgebildet werden können.

Figur 2 zeigt schematisch eine Schnittansicht der Platte 60 längs Linie 2-2 mit dem ausgeschnittenen Instrumentenkörperteilrohling 58.

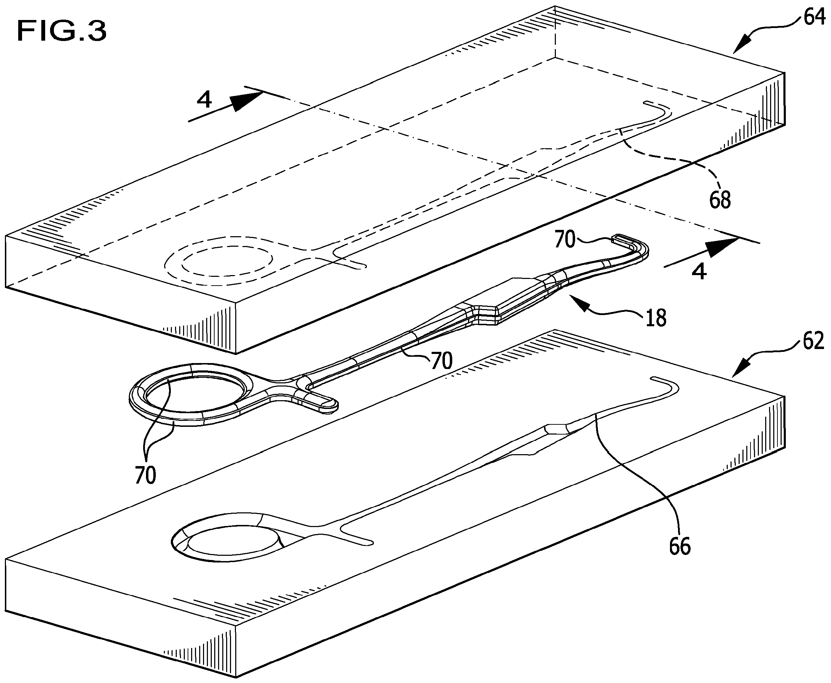

Der Instrumentenkörperteilrohling 58 wird zur Ausbildung eines der Instrumentenkörperteile 18 und 20 umgeformt. Figur 3 zeigt schematisch zwei Formwerkzeuge 62 und 64 mit jeweils einer einer Fertigkontur entsprechenden Aufnahme 66 beziehungsweise 68 zur Ausbildung einer oberen und einer unteren Hälfte des Instrumentenkörperteils 18 beziehungsweise 20.

Der Instrumentenkörperteilrohling 58 wird in die Aufnahmen 66 und 68 der Formwerkzeuge 62 und 64 eingesetzt und die Formwerkzeuge 62 und 64 werden dann gegeneinander gepresst. Durch das beschriebene Kaltmassivumformen des aus einem metallischen Werkstoff ausgebildeten Instrumentenkörperteilrohlings 58 wird der Instrumentenkörperteil 18 ausgebildet und weist im Wesentlichen seine Fertigkontur auf.

Die Formwerkzeuge 62 und 64 sind so ausgebildet, dass sie durch das Zusammenpressen das Instrumentenkörperteil 18 mit allseitig vorstehenden Graten 70 ausbilden. Die Grate 70 weisen parallel zur Bearbeitungsebene 36.

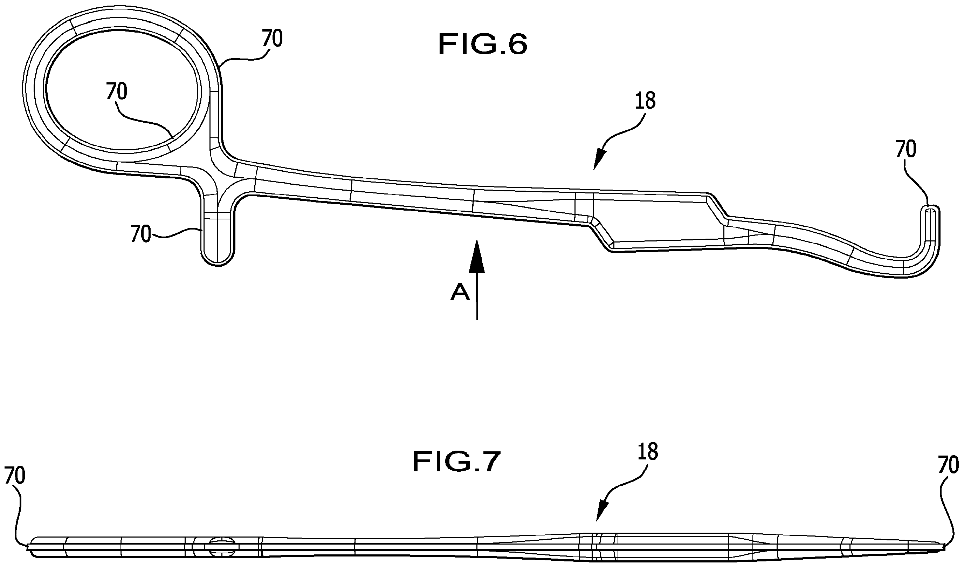

Das Kaltmassivumformen wird vorzugsweise so durchgeführt, dass die ausgebildeten Grate 70 in sich geschlossen sind. In Figur 6 sind beispielshaft bei dem Instrumentenkörperteil 18 zwei in sich geschlossen ausgebildete Grate 70 schematisch dargestellt.

Die überstehenden Grate 70 werden entfernt, und zwar durch Fräsen. Hierzu wird der Instrumentenkörperteil 18 maschinell bearbeitet. Ein Fräswerkzeug 72 wird von einem Antrieb 74 um eine Entgratungslängsachse 76 rotiert. Der Antrieb 74 wird von einer mit ihm steuerungswirksam verbundenen Steuerungseinrichtung 78 angesteuert, um den Antrieb 74 mit dem Fräswerkzeug 72 in gewünschter Weise zu bewegen. Der Antrieb 74 bewegt das Fräswerkzeug 72 am Instrumentenkörperteil 18 in definiert vorgegebener Weise entlang, um die vorstehenden Grate 70 zu entfernen.

Beim Entgraten wird das Fräswerkzeug 72 derart bewegt, dass die Entgratungslängsachse stets senkrecht zur Bearbeitungsebene 36 orientiert ist. Die Entgratungslängsachse 76 behält während des gesamten Entgratungsprozesses diese Orientierung relativ zur Bearbeitungsebene 36 bei.

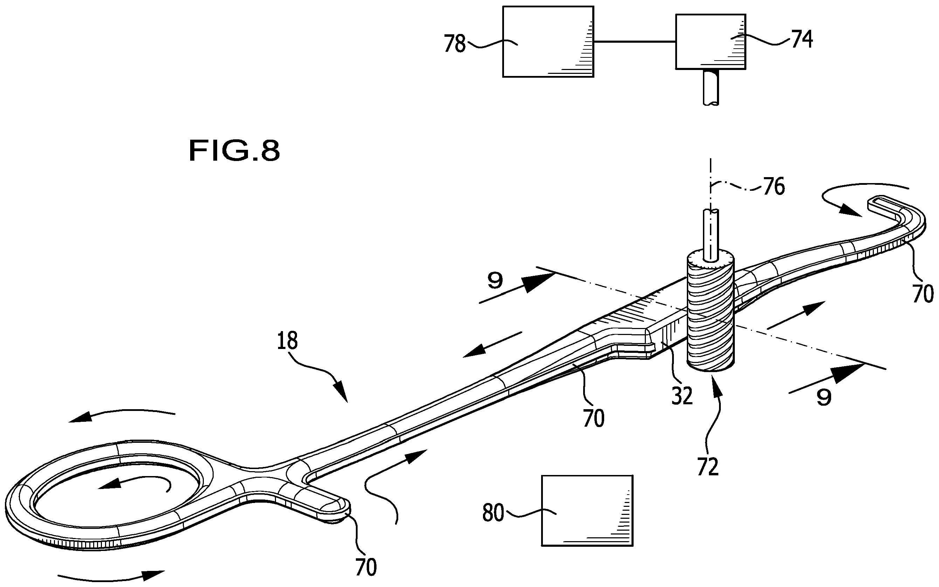

Beim Entgraten ist das Instrumentenkörperteil 18 mit einer Halteeinrichtung 80, die in Figur 8 schematisch dargestellt ist, gehalten. Auf diese Weise kann eine Orientierung oder Ausrichtung der Entgratungslängsachse 76 zum Instrumentenkörperteil 18 auf einfache Weise beibehalten werden.

Beim Entfernen der Grate 70 mit dem Fräswerkzeuge 72 werden nicht nur die Grate 70 entfernt, sondern auch ein Teil des Instrumentenkörperteils 18 zur Ausbildung der Entgratungsflächen 32 und 34, die in der beschriebenen Weise senkrecht zur Bearbeitungsebene 36 orientiert sind.

Das mit der Halteeinrichtung 80 gehaltene Instrumentenkörperteil 18 wird im Bereich des Schlusskastens 24 mit der senkrecht zur Bearbeitungsebene 36 orientierte Gelenkbohrung 28 versehen. Ferner wird der parallel zur Bearbeitungsebene 36 orientierte Schlitz 22 ausgebildet.

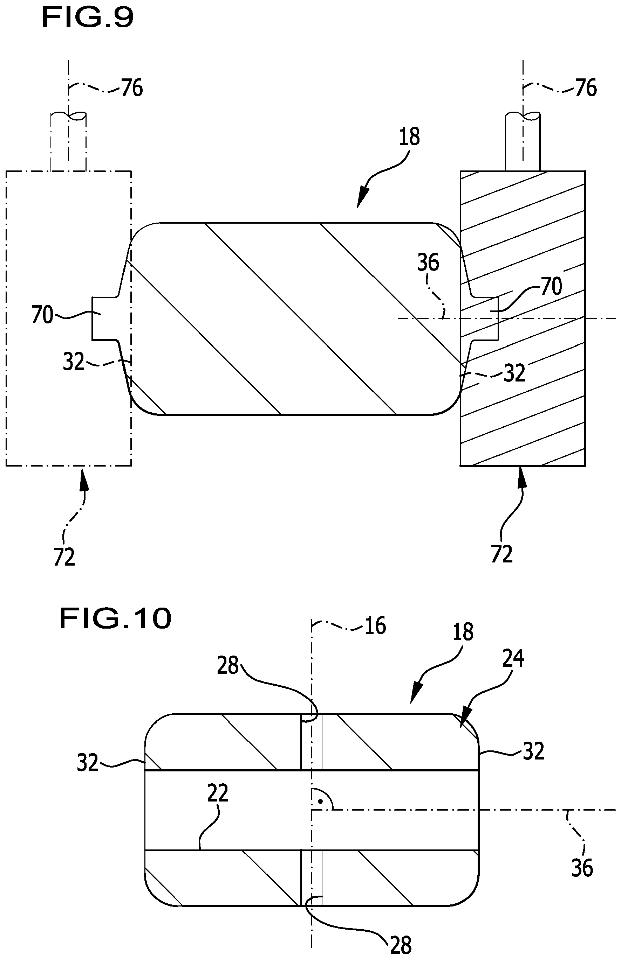

Wie insbesondere in Figur 10 gut zu erkennen, verlaufen die Entgratungsflächen 32 parallel zur Schwenkachse 16. Dies folgt unmittelbar aus der Orientierung des Fräswerkzeugs 72 mit seiner Entgratungslängsachse 76.

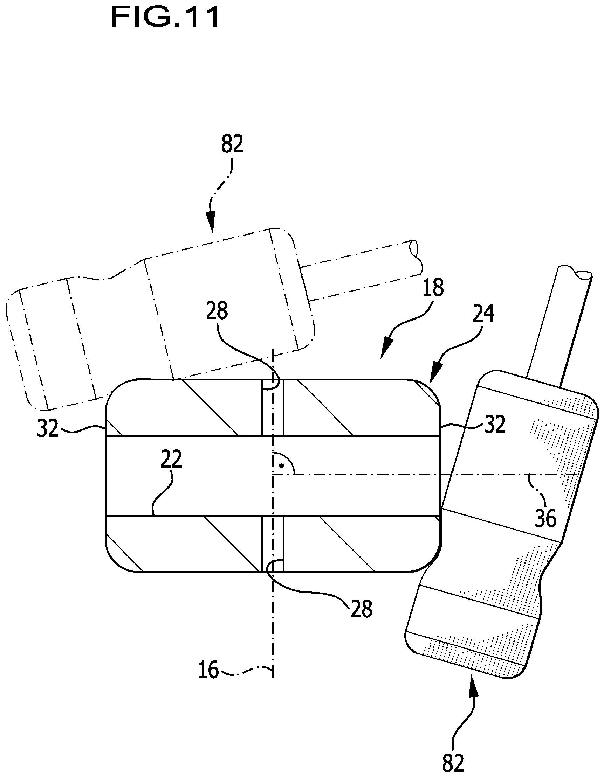

Das Instrumentenkörperteil 18 kann optional, wie schematisch in Figur 11 dargestellt, poliert werden. Figur 11 zeigt hierzu beispielhaft ein Polierwerkzeug 82, mit dem nicht verrundete Übergänge am Instrumentenkörperteil 18, die durch das Entgraten entstehen, verrundet werden.

Alternativ zum Polieren mit dem Polierwerkzeug 82 können die entgrateten Instrumentenkörperteile 18 auch durch Gleitschleifen in einer Gleitschleifanlage poliert werden.

Abschließend können die Instrumentenkörperteile 18 und 20 noch durch Strahlen behandelt werden, beispielsweise durch Sandstrahlen oder Kugelstrahlen. So lässt sich eine definierte und ästhetisch ansprechende Oberfläche der Instrumentenkörperteile 18 und 20 realisieren.

Die wie beschrieben ausgebildeten Instrumentenkörperteile 18 und 20 können dann wie eingangs erläutert mit dem Gelenkstift 26 zur Ausbildung des Instruments 10 verbunden werden.

In der beschriebenen Weise lassen sich unterschiedlichste medizinische Instrumenten ausbilden, beispielsweise Scheren, Klemmen, Nadelhalter oder Zangen. Die Instrumente werden für den jeweiligen Einsatzzweck mit entsprechenden Werkzeugelementen ausgebildet.

Das beschriebene Herstellungsverfahren ermöglicht eine nahezu vollständige Herstellung von Instrumenten 10 rein maschinell. Insbesondere können CNC-Fräsen zum Entgraten der Instrumentenkörperteile 18 und 20 nach dem Umformen aus Instrumentenkörperrohlingen 58 eingesetzt werden. Dies ermöglicht eine hohe Reproduzierbarkeit und damit eine gleichbleibend hohe Qualität der Instrumente 10.

Das beschriebene Herstellungsverfahren ermöglicht insbesondere eine sichere Identifizierung von Instrumenten 10, die nach dem beschriebenen Verfahren ausgebildet sind. Die Ausrichtung der Schwenkachse 16 relativ zu den Entgratungsflächen 32 und 34 kann aufgrund der maschinellen Bearbeitung hochpräzise erreicht werden, was insbesondere durch handgeführte Bearbeitungsschritte wie handgeführtes Bandschleifen der umgeformten Instrumentenkörperteilrohlinge 58 zum Entfernern der Grate 70 gerade nicht möglich ist.

Das Entfernen der Grate 70 wurde oben mit einem Fräswerkzeug 72 beschrieben, das in Form eines Umfangsfräsers ausgebildet ist. Grundsätzlich ist es auch möglich, die Entgratungsflächen 32 und 34 mit einem Fräswerkzeug 62 in Form eines Stirnfräsers auszubilden. Ein Stirnfräser ist dann jedoch mit der Entgratungslängsachse 76 nicht senkrecht zur Bearbeitungsebene 36 orientiert, sondern parallel zu dieser. Er wird also um eine Achse rotiert, die parallel zur Bearbeitungsebene 36 verläuft, insbesondere in dieser liegt. In diesem Fall kann jedoch ebenfalls eine Entgratungslängsachse 76 definiert werden, zu der alle Entgratungsflächen 32 und 34 am Instrument 10 parallel verlaufen und die senkrecht zur Bearbeitungsebene 36 orientiert ist.

### Bezugszeichenliste

- 10: Medizinisches Instrument
- 12: Tuchklemme
- 14: Schlussbereich
- 16: Schwenkachse
- 18: Instrumentenkörperteil
- 20: Instrumentenkörperteil
- 22: Schlitz
- 24: Schlusskasten
- 26: Gelenkstift
- 28: Gelenkbohrung
- 30: Schlussabschnitt
- 32: Entgratungsfläche
- 34: Entgratungsfläche
- 36: Bearbeitungsebene
- 38: Normale
- 40: Werkzeugelement
- 42: Werkzeugelement
- 44: Branche
- 46: Branche
- 48: Fingerring
- 50: Fingerring
- 52: Sperrelement
- 54: Sperrelement
- 56: Sperreinrichtung
- 58: Instrumentenkörperteilrohling
- 60: Platte
- 62: Formwerkzeug
- 64: Formwerkzeug
- 66: Aufnahme
- 68: Aufnahme
- 70: Grat
- 72: Fräswerkzeug
- 74: Antrieb
- 76: Entgratungslängsachse
- 78: Steuerungseinrichtung
- 80: Halteeinrichtung
- 82: Polierwerkzeug
- 84: Flächenabschnitt
- 86: Flächenabschnitt

## Patentansprüche

1. Medizinisches Instrument (10) mit mindestens einem Instrumentenkörperteil (18, 20), wobei der mindestens eine Instrumentenkörperteil (18, 20) aus einem Instrumentenkörperteilrohling (58) durch Umformen ausgebildet ist, **dadurch gekennzeichnet, dass** das mindestens eine Instrumentenkörperteil (18, 20) mindestens eine durch Fräsen, insbesondere durch maschinelles Fräsen, ausgebildete Entgratungsfläche (32, 34) aufweist, dass die mindestens eine Entgratungsfläche (32, 34) einen oder mehrere Abschnitte umfasst, dass jeder Abschnitt der mindestens einen Entgratungsfläche (32, 34) senkrecht zu einer gemeinsamen Bearbeitungsebene (36) verläuft, dass eine Normale (38) zur Bearbeitungsebene (36) eine Entgratungslängsachse (76) definiert, dass die mindestens eine Entgratungsfläche (32, 34) parallel zur Entgratungslängsachse (76) verläuft, das medizinische Instrument (10) in Form einer Schere, einer Zange, eines Nadelhalters oder einer Klemme ausgebildet ist und zwei Instrumentenkörperteile (18, 20) umfasst, dass die zwei Instrumentenkörperteile (18, 20) um eine Schwenkachse (16) verschwenkbar aneinander gelagert sind und dass die Schwenkachse (16) die Entgratungslängsachse (76) definiert.

2. Medizinisches Instrument nach Anspruch 1, **dadurch gekennzeichnet, dass** die mindestens eine Entgratungsfläche (34) in sich geschlossen ausgebildet ist.

3. Medizinisches Instrument nach einem der voranstehenden Ansprüche, **dadurch gekennzeichnet, dass** die mindestens eine Entgratungsfläche (32, 34) abschnittsweise eben und/oder abschnittsweise vom mindestens einen Instrumentenkörperteil (18, 20) weg weisend konvex gekrümmt und/oder vom mindestens einen Instrumentenkörperteil (18, 20) weg weisend konkav gekrümmt ausgebildet ist.

4. Medizinisches Instrument nach Anspruch 3, **dadurch gekennzeichnet, dass** die mindestens eine abschnittsweise gekrümmte Entgratungsfläche (32, 34) nur eindimensional gekrümmt ausgebildet ist und dass ein Krümmungsradius der Entgratungsfläche (32, 34) ausschließlich bezogen auf die Entgratungslängsachse (76) definiert ist.

5. Medizinisches Instrument nach Anspruch 4, **dadurch gekennzeichnet, dass** der Krümmungsradius abschnittsweise konstant ist oder sich längs der Erstreckung der mindestens einen Entgratungsfläche (32, 34) sich abschnittsweise oder kontinuierlich ändert.

6. Medizinisches Instrument nach einem der voranstehenden Ansprüche, **dadurch gekennzeichnet, dass** mindestens zwei Flächenabschnitte (84, 86) der mindestens einen Entgratungsfläche (32, 34) parallel oder im Wesentlichen parallel zueinander verlaufen.

7. Medizinisches Instrument nach einem der voranstehenden Ansprüche, **dadurch gekennzeichnet, dass** das mindestens eine Instrumentenkörperteil (18, 20)
a) durch Kaltmassivumformen aus dem Instrumentenkörperteilrohling (58) ausgebildet ist
und/oder
b) poliert ist, insbesondere durch Gleitschleifen,
und/oder
c) gestrahlt ist, insbesondere sandgestrahlt oder kugelgestrahlt, und/oder
d) aus einem metallischen Werkstoff, insbesondere aus einem Instrumentenstahl, ausgebildet ist.

8. Verfahren zum Herstellen eines medizinischen Instruments (10), welches mindestens ein Instrumentenkörperteil (18, 20) umfasst, bei welchem Verfahren ein Instrumentenkörperteilrohling (58) umgeformt wird und beim Umformen, insbesondere durch Toleranz- und Prozessschwankungen, ausgebildete Grate (70) entfernt werden, **dadurch gekennzeichnet, dass** die Grate (70) durch Fräsen, insbesondere maschinelles Fräsen, entfernt werden, dass das mindestens eine Instrumentenkörperteil (18, 20) beim Fräsen gehalten wird, dass ein Fräswerkzeug (72) zum Entfernen der Grate (70) um eine Entgratungslängsachse (76) rotiert und zum Ausbilden von mindestens einer Entgratungsfläche (32, 34) in einer Bearbeitungsebene (36), deren Normale (38) die Entgratungslängsachse (76) definiert, bewegt wird ohne eine Ausrichtung der Entgratungslängsachse (76) relativ zur Bearbeitungsebene (36) beim Entgraten zu verändern, dass am mindestens einen Instrumentenkörperteil (18, 20) eine Gelenkbohrung (28) ausgebildet wird mit einer Bohrungslängsachse (16), die parallel oder im Wesentlichen parallel zur Entgratungslängsachse (76) verläuft und dass mindestens zwei, insbesondere nur zwei, Instrumentenkörperteile (18, 20) miteinander um die Bohrungslängsachse (16) verschwenkbar miteinander gekoppelt werden und dass das medizinische Instrument (10) in Form einer Schere, einer Zange, eines Nadelhalters oder einer Klemme ausgebildet wird.

9. Verfahren nach Anspruch 8, **dadurch gekennzeichnet, dass** das Entfernen der Grate (70) computergesteuert durchgeführt wird.

10. Verfahren nach Anspruch 8 oder 9, **dadurch gekennzeichnet, dass** beim Entfernen der Grate (70) ein Teil des Instrumentenkörperteils (18) zur Ausbildung der mindestens einen Entgratungsfläche (32, 34) mit entfernt wird.

11. Verfahren nach einem der Ansprüche 8 bis 10, **dadurch gekennzeichnet, dass** der Instrumentenkörperteilrohling (58)
a) aus einer Platte (60) durch Stanzen oder Schneiden, insbesondere Laserschneiden, ausgebildet wird
und/oder
b) aus einem metallischen Werkstoff, insbesondere aus einem Instrumentenstahl, ausgebildet wird
und/oder
c) durch Kaltmassivumformen umgeformt wird
und/oder
d) auf eine Fertigkontur des mindestens einen Instrumentenkörperteils (18, 20) umgeformt wird, wobei die ausgebildeten Grate (70) über die Fertigkontur vorstehen.

12. Verfahren nach einem der Ansprüche 8 bis 11, **dadurch gekennzeichnet, dass** beim Umformen des Instrumentenkörperteilrohlings (58) mindestens ein zusammenhängender Grat (70) ausgebildet wird.

13. Verfahren nach einem der Ansprüche 8 bis 12, **dadurch gekennzeichnet, dass** zum verschwenkbaren Koppeln der zwei Instrumentenkörperteile (18, 20) ein gemeinsamer Gelenkstift (26) in die Gelenkbohrungen (28) beider Instrumentenkörperteile (18, 20) eingesetzt und mit einem der beiden Instrumentenkörperteile (18, 20) dauerhaft verbunden wird, insbesondere kraft- und/oder form- und/oder stoffschlüssig, weiter insbesondere durch Kleben oder Schweißen.

14. Verfahren nach einem der Ansprüche 8 bis 13, **dadurch gekennzeichnet, dass** das mindestens eine Instrumentenkörperteil (18, 20) poliert wird, insbesondere durch Gleitschleifen,
wobei das mindestens eine Instrumentenkörperteil (18, 20)
a) nach dem Entfernen aller Grate (70) poliert wird und und/üde bevorzugt
b) nach dem Polieren gestrahlt wird, insbesondere durch Sandstrahlen oder Kugelstrahlen.

15. Verwendung eines Verfahrens nach einem der Ansprüche 8 bis 14 zum Herstellen eines medizinischen Instruments (10) nach einem der Ansprüche 1 bis 7.

## Claims

1. Medical instrument (10) with at least one instrument body part (18, 20), wherein the at least one instrument body part (18, 20) is made from an instrument body part blank (58) by forming, **characterized in that** the at least one instrument body part (18, 20) has at least one deburring face (32, 34) formed by milling, in particular by machine milling, **in that** the at least one deburring face (32, 34) comprises one or more portions, **in that** each portion of the at least one deburring face (32, 34) extends perpendicularly to a common machining plane (36), **in that** a normal (38) to the machining plane (36) defines a deburring longitudinal axis (76), **in that** the at least one deburring face (32, 34) extends in parallel to the deburring longitudinal axis (76), the medical instrument (10) is configured in the form of scissors, pliers, a needle holder or a clamp and comprises two instrument body parts (18, 20), **in that** the two instrument body parts (18, 20) are mounted on one another so as to be pivotable about a pivot axis (16), and **in that** the pivot axis (16) defines the deburring longitudinal axis (76).

2. Medical instrument in accordance with Claim 1, **characterized in that** the at least one deburring face (34) is of self-enclosed configuration.

3. Medical instrument in accordance with any one of the preceding Claims, **characterized in that** the at least one deburring face (32, 34) is configured to be planar in sections and/or convexly curved facing away from the at least one instrument body part (18, 20) in sections and/or concavely curved facing away from the at least one instrument body part (18, 20).

4. Medical instrument in accordance with Claim 3, **characterized in that** the at least one deburring face (32, 34) that is curved in sections is configured to be curved in only one dimension, and **in that** a radius of curvature of the deburring face (32, 34) is defined exclusively in relation to the deburring longitudinal axis (76).

5. Medical instrument in accordance with Claim 4, **characterized in that** the radius of curvature is constant in sections or changes in sections or continuously along the extent of the at least one deburring face (32, 34).

6. Medical instrument in accordance with any one of the preceding Claims, **characterized in that** at least two face portions (84, 86) of the at least one deburring face (32, 34) extend in parallel or substantially in parallel to one another.

7. Medical instrument in accordance with any one of the preceding Claims, **characterized in that** the at least one instrument body part (18, 20)
a) is formed from the instrument body part blank (58) by cold forming and/or
b) is polished, in particular by vibratory grinding,
and/or
c) is blasted, in particular sandblasted or shot peened,
and/or
d) is made of a metallic material, in particular of an instrument steel.

8. Method for producing a medical instrument (10), which comprises at least one instrument body part (18, 20), in which method an instrument body part blank (58) is reshaped and burrs (70) that were created during forming, in particular due to tolerance and process fluctuations, are removed, **characterized in that** the burrs (70) are removed by milling, in particular machine milling, **in that** the at least one instrument body part (18, 20) is held during milling, **in that** a milling tool (72), for the purpose of removing the burrs (70), is rotated about a deburring longitudinal axis (76) and, for the purpose of creating at least one deburring face (32, 34), is moved in a machining plane (36), the normal (38) of which defines the deburring longitudinal axis (76), without changing an alignment of the deburring longitudinal axis (76) relative to the machining plane (36) during deburring, **in that** formed on the at least one instrument body part (18, 20) is a joint bore (28) with a bore longitudinal axis (16), said axis extending in parallel or substantially in parallel to the deburring longitudinal axis (76), and **in that** at least two, in particular only two, instrument body parts (18, 20) are coupled to one another so as to be pivotable with one another about the bore longitudinal axis (16), and **in that** the medical instrument (10) is made in the form of scissors, pliers, a needle holder or a clamp.

9. Method in accordance with Claim 8, **characterized in that** the removal of the burrs (70) is performed in a computer-controlled manner.

10. Method in accordance with Claim 8 or 9, **characterized in that** during removal of the burrs (70), a part of the instrument body part (18) is also removed for creating the at least one deburring face (32, 34).

11. Method in accordance with any one of Claims 8 to 10, **characterized in that** the instrument body part blank (58)
a) is made from a plate (60) by punching or cutting, in particular laser cutting,
and/or
b) is made of a metallic material, in particular of an instrument steel, and/or
c) is reshaped by cold forming
and/or
d) is reshaped to a final contour of the at least one instrument body part (18, 20), the burrs (70) that are created projecting beyond the final contour.

12. Method in accordance with any one of Claims 8 to 11, **characterized in that** at least one contiguous burr (70) is created during the forming of the instrument body part blank (58).

13. Method in accordance with any one of Claims 8 to 12, **characterized in that** for pivotably coupling the two instrument body parts (18, 20), a common hinge pin (26) is inserted into the joint bores (28) of both instrument body parts (18, 20) and is permanently connected to one of the two instrument body parts (18, 20), in particular in a force- and/or positive-locking and/or materially bonded manner, further particularly by adhesion or welding.

14. Method in accordance with any one of Claims 8 to 13, **characterized in that** the at least one instrument body part (18, 20) is polished, in particular by vibratory grinding,
wherein the at least one instrument body part (18, 20)
a) is polished after the removal of all burrs (70)
and, preferably,
b) is blasted, in particular by sandblasting or shot peening, after polishing.

15. Use of a method in accordance with any one of Claims 8 to 14 for producing a medical instrument (10) in accordance with any one of Claims 1 to 7.

## Revendications

1. Instrument médical (10) avec au moins une partie de corps d'instrument (18, 20), dans lequel la au moins une partie de corps d'instrument (18, 20) est réalisée à partir d'une ébauche de partie de corps d'instrument (58) par formage, **caractérisé en ce que** la au moins une partie de corps d'instrument (18, 20) présente au moins une surface d'ébavurage (32, 34) réalisée par fraisage, en particulier par fraisage mécanique, que la au moins une surface d'ébavurage (32, 34) comprend une ou plusieurs sections, que chaque section de la au moins une surface d'ébavurage (32, 34) s'étend perpendiculairement à un plan d'usinage (36) commun, qu'une normale (38) au plan d'usinage (36) définit un axe longitudinal d'ébavurage (76), que la au moins une surface d'ébavurage (32, 34) s'étend parallèlement à l'axe longitudinal d'ébavurage (76), l'instrument médical (10) est réalisé sous forme de cisailles, d'une pince, d'un porte-aiguilles ou d'un élément de serrage et comprend deux parties de corps d'instrument (18, 20), que les deux parties de corps d'instrument (18, 20) sont montées l'une sur l'autre de manière à pouvoir pivoter autour d'un axe de pivotement (16) et que l'axe de pivotement (16) définit l'axe longitudinal d'ébavurage (76).

2. Instrument médical selon la revendication 1, **caractérisé en ce que** la au moins une surface d'ébavurage (34) est réalisée de manière fermée sur elle-même.

3. Instrument médical selon l'une quelconque des revendications précédentes, **caractérisé en ce que** la au moins une surface d'ébavurage (32, 34) est réalisée sur certaines sections de manière plane et/ou sur certaines sections de manière courbée de façon convexe en s'éloignant d'au moins une partie de corps d'instrument (18, 20) et/ou de manière courbée de façon concave en s'éloignant d'au moins une partie de corps d'instrument (18, 20).

4. Instrument médical selon la revendication 3, **caractérisé en ce que** la au moins une surface d'ébavurage (32, 34) courbée sur certaines sections est réalisée de manière courbée uniquement de façon unidimensionnelle et qu'un rayon de courbure de la surface d'ébavurage (32, 34) est défini exclusivement par rapport à l'axe longitudinal d'ébavurage (76).

5. Instrument médical selon la revendication 4, **caractérisé en ce que** le rayon de courbure est constant sur certaines sections ou varie le long de l'étendue de la au moins une surface d'ébavurage (32, 34) sur certaines sections ou en continu.

6. Instrument médical selon l'une quelconque des revendications précédentes, **caractérisé en ce qu'**au moins deux sections de surface (84, 86) de la au moins une surface d'ébavurage (32, 34) s'étendent parallèlement ou sensiblement parallèlement l'une à l'autre.

7. Instrument médical selon l'une quelconque des revendications précédentes, **caractérisé en ce que** la au moins une partie de corps d'instrument (18, 20)
a) est réalisée par matriçage à froid à partir de l'ébauche de partie de corps d'instrument (58)
et/ou
b) est polie, en particulier par tribofinition,
et/ou
c) subit un nettoyage de surface, en particulier est sablée ou grenaillée,
et/ou
d) est réalisée à partir d'un matériau métallique, en particulier à partir d'un acier pour instruments médicaux.

8. Procédé pour fabriquer un instrument médical (10), lequel comprend au moins une partie de corps d'instrument (18, 20), procédé selon lequel une ébauche de partie de corps d'instrument (58) est formée et des bavures (70) réalisées lors du formage, en particulier dues à des fluctuations de tolérance et de processus, sont retirées, **caractérisé en ce que** les bavures (70) sont retirées par fraisage, en particulier fraisage mécanique, que la au moins une partie de corps d'instrument (18, 20) est retenue lors du fraisage, qu'un outil de fraisage (72) pour retirer les bavures (70) tourne autour d'un axe longitudinal d'ébavurage (76) et pour réaliser au moins une surface d'ébavurage (32, 34) est déplacé dans un plan d'usinage (36), dont la normale (38) définit l'axe longitudinal d'ébavurage (76), sans modifier une orientation de l'axe longitudinal d'ébavurage (76) par rapport au plan d'usinage (36) lors de l'ébavurage, qu'un trou d'articulation (28) est réalisé sur la au moins une partie de corps d'instrument (18, 20) avec un axe longitudinal de trou (16), qui s'étend parallèlement ou sensiblement parallèlement à l'axe longitudinal d'ébavurage (76) et qu'au moins deux, en particulier uniquement deux, parties de corps d'instrument (18, 20) sont accouplées l'une à l'autre de manière à pouvoir pivoter autour de l'axe longitudinal de trou (16) et que l'instrument médical (10) est réalisé sous forme de cisailles, d'une pince, d'un porte-aiguilles ou d'un élément de serrage.

9. Procédé selon la revendication 8, **caractérisé en ce que** le retrait des bavures (70) est mis en oeuvre de manière commandée par ordinateur.

10. Procédé selon la revendication 8 ou 9, **caractérisé en ce que** lors du retrait des bavures (70) une partie de la partie de corps d'instrument (18) est retirée pour la réalisation de la au moins une surface d'ébavurage (32, 34).

11. Procédé selon l'une quelconque des revendications 8 à 10, **caractérisé en ce que** l'ébauche de partie de corps d'instrument (58)
a) est réalisée à partir d'une plaque (60) par estampage ou découpage, en particulier découpage laser,
et/ou
b) est réalisée à partir d'un matériau métallique, en particulier à partir d'un acier pour instruments chirurgicaux,
et/ou
c) est formée par matriçage à froid
et/ou
d) est formée à un contour fini de la au moins une partie de corps d'instrument (18, 20), dans lequel les bavures (70) réalisées font saillie du contour fini.

12. Procédé selon l'une quelconque des revendications 8 à 11, **caractérisé en ce que** lors du formage de l'ébauche de partie de corps d'instrument (58) au moins une bavure (70) d'un seul tenant est réalisée.

13. Procédé selon l'une quelconque des revendications 8 à 12, **caractérisé en ce que** pour accoupler de manière pivotante les deux parties de corps d'instrument (18, 20) une tige d'articulation (26) commune est insérée dans les trous d'articulation (28) des deux parties de corps d'instrument (18, 20) et reliée de manière permanente à l'une des deux parties de corps d'instrument (18, 20), en particulier à force et/ou par complémentarité de formes et/ou par liaison de matière, plus particulièrement par collage ou soudage.

14. Procédé selon l'une quelconque des revendications 8 à 13, **caractérisé en ce que** la au moins une partie de corps d'instrument (18, 20) est polie, en particulier par tribofinition,
dans lequel la au moins une partie de corps d'instrument (18, 20)
a) est polie après le retrait de toutes les bavures (70) et de préférence
b) subit un traitement de surface après le polissage, en particulier par sablage ou grenaillage.

15. Utilisation d'un procédé selon l'une quelconque des revendications 8 à 14 pour fabriquer un instrument médical (10) selon l'une quelconque des revendications 1 à 7.
